# EUROPÄISCHE PATENTSCHRIFT

(11) **EP 1 967 132 B1**
(45) Veröffentlichungstag und Bekanntmachung des Hinweises auf die Patenterteilung: **04.03.2015**
(21) Anmeldenummer: 07004859.0
(22) Anmeldetag: 09.03.2007
(51) Int. Cl.: A61B 5/00

(54) **Medizinisches Datenübertragungssystem**
Medical data transmission system
Système médical de transmission des données

(43) Veröffentlichungstag der Anmeldung: 10.09.2008
(73) Patentinhaber: Roche Diagnostics GmbH, 68305 Mannheim (DE); F.Hoffmann-La Roche AG, 4070 Basel (CH)
(72) Erfinder: Bruegger, Martin, CH-3098 Köniz (CH); Oberli, Markus, 3422 Kirchberg (CH); Lindegger, Stefan, 4932 Lotzwil (CH); Roesicke, Bernd, 68305 Mannheim (CH)
(74) Vertreter: Twelmeier Mommer & Partner

(56) Entgegenhaltungen:
- EP-A2- 0 978 254
- EP-A2- 1 062 985
- WO-A-2006/102086
- US-A1- 2003 130 590

## Beschreibung

Die Erfindung geht aus von einem medizinischen Datenübertragungssystem mit den im Oberbegriff des Anspruchs 1 angegebenen Merkmalen, wie es aus der WO 2006/133851 A2 sowie der US 4883064 bekannt ist.

Zu einem derartigen Datenübertragungssystem gehören mindestens zwei Geräte. Ein erstes Gerät wird bestimmungsgemäß von einem Patienten am Körper getragen und erzeugt im Betrieb medizinisch relevante Daten, die drahtlos zu einem zweiten Gerät übertragen werden. Bei dem ersten Gerät handelt es sich typischerweise um ein Messgerät, das mit einem Sensor im oder am Körper eines Patienten Messungen vornimmt und als medizinisch relevante Daten Messdaten erzeugt, die zu dem zweiten Gerät übertragen werden. Bei dem ersten Gerät kann es sich aber auch um ein Behandlungsgerät handeln, beispielsweise ein Infusionsgerät, das einen medizinischen Wirkstoff, insbesondere Insulin, verabreicht. Bei den medizinisch relevanten Daten, die von einem solchen Behandlungsgerät erzeugt werden, handelt es sich typischerweise um Behandlungsdaten, beispielsweise Infusionsraten oder ähnliches.

Das zweite Gerät eines derartigen medizinischen Datenübertragungssystems dient typischerweise zur Anzeige, Auswertung der von dem ersten Gerät erzeugten medizinisch relevanten Daten und/oder der Steuerung des ersten Geräts.

In dem ersten Gerät werden die medizinisch relevanten Daten von einer Schaltung erzeugt, die beispielsweise einen Sensor zur in-vivo Messung einer Analytkonzentration enthält und von einer internen Kommunikationseinheit drahtlos zu dem zweiten Gerät übertragen werden. Die von der Schaltung erzeugten Daten werden dabei zunächst in einem Übergabespeicher abgelegt, aus dem sie von der Kommunikationseinheit ausgelesen werden können. Notwendigerweise müssen deshalb sowohl die datenerzeugende Schaltung als auch die Kommunikationseinheit auf den Übergabespeicher zugreifen können. Zur Vermeidung von Kollisionen werden deshalb bei bekannten Datenübertragungssystemen mit Direktzugriff (random access) auf Daten Zugriffsregeln und Kontrollmechanismen definiert.

Die WO 2006/133851 A2 lehrt bei einem medizinischen Datenübertragungssystem, einen Übergabespeicher zu verwenden, der sowohl für die Schaltung als auch für die Kommunikationseinheit jeweils einen eigenen Dateneingang und Datenausgang aufweist. Ein gleichzeitiger Zugriff der Schaltung und der Kommunikationseinheit auf den Übergabespeicher ist zwar unwahrscheinlich, kann jedoch insbesondere bei Verwendung von vorgegebenen Protokollen zur Kommunikation mit dem zweiten Gerät, beispielsweise Bluetooth, zu erheblichen Problemen führen.

Bei asynchronen Kommunikationsprozessen mit Zugriff verschiedener Systemkomponenten auf einen gemeinsamen Speicher ist deshalb generell mit einer erhöhten Wahrscheinlichkeit einer Datenkorruption zu rechen. Dies macht einen hoher Aufwand zur Datensicherung, beispielsweise die Verwendung von Pufferspeichern zur kurzfristigen Zwischenspeicherung von Daten, und zusätzlich ein hohes Maß an Absprache und Kontrolle zwischen den beteiligten Systemkomponenten erforderlich.

Aus der US 6,571,128 B2 ist es bekannt, bei einem medizinischen Datenübertragungssystem Zugriffskonflikte zwischen der datenerzeugenden Schaltung und der Kommunikationseinheit auf einen gemeinsamen Speicher dadurch zu vermeiden, dass die datenerzeugende Schaltung einen Mikroprozessor als Master und die Kommunikationseinheit ein weiterer Mikroprozessor als Slave enthalten. Auf diese Weise können Zugriffskonflikte zwar ausgeschlossen werden, jedoch ist der Zugriff der Kommunikationseinheit auf den Übergabespeicher nur noch eingeschränkt möglich. Als weiterer Nachteil kommt hinzu, dass bei einem Defekt des Masterprozessors der Mikroprozessor der Kommunikationseinheit in der Regel gar nicht mehr auf den Übergabespeicher zugreifen kann und folglich alle auf dem Übergabespeicher gespeicherten Daten verloren sind. Dies ist besonders schwerwiegend, wenn Daten nur recht selten, beispielsweise im Abstand von etwa Woche, von dem ersten Gerät zu dem zweiten Gerät übertragen werden.

Aufgabe der Erfindung ist es deshalb, einen Weg aufzuzeigen, wie bei einem medizinischen Datenübertragungssystem die Zugriffsmöglichkeiten der Kommunikationseinheit auf einen Übergabespeicher verbessert werden können ohne den Abstimmungs- und Steuerungsaufwand zwischen den beteiligten Systemkomponenten zu erhöhen.

Diese Aufgabe wird erfindungsgemäß durch ein medizinisches Datenübertragungssystem mit den im Anspruch 1 angegebenen Merkmalen gelöst. Vorteilhafte Weiterbildungen der Erfindung sind Gegenstand der Unteransprüche.

Bei einem erfindungsgemäßen Datenübertragungssystem ist keine Synchronisation oder Abstimmung zwischen der Daten erzeugenden Schaltung und der Kommunikationseinheit erforderlich. Da folglich auch keine Steuerungssignale zwischen der Daten erzeugenden Schaltung und der Kommunikationseinheit ausgetauscht werden müssen, lässt sich ein erfindungsgemäßes System mit einem vorteilhaft geringen Aufwand hinsichtlich Hardware, Protokollsoftware, Projektorganisation und Evaluierung realisieren.

Bei einem erfindungsgemäßen Datenübertragungssystem ist die datenerzeugende Schaltung über eine erste Datenleitung und die Kommunikationseinheit über eine zweite Datenleitung an den Übergabespeicher angeschlossen. Ein Umschalter schließt in einem ersten Schaltzustand die erste Datenleitung und unterbricht die zweite Datenleitung, so dass in dem ersten Schaltzustand ausschließlich die datenerzeugende Schaltung auf den Übergabespeicher zugreifen kann. In einem zweiten Schaltzustand des Umschalters ist die erste Datenleitung unterbrochen und die zweite Datenleitung geschlossen, so dass ausschließlich die Kommunikationseinheit auf den Übergabespeicher zugreifen kann. Der Umschalter wechselt dabei ohne Kommunikation mit der datenerzeugenden Schaltung zwischen dem ersten und dem zweiten Schaltzustand. Auf diese Weise kann jede Einflussnahme der datenerzeugenden Schaltung auf die Kommunikationseinheit ausgeschlossen werden und somit eine echte Alternative zu bekannten Master-Slave-Architekturen geschaffen werden. Insbesondere ist bei einem erfindungsgemäßen Datenübertragungssystem der Zugriff der Kommunikationseinheit auf den Übergabespeicher und damit auf die medizinisch relevanten Daten, die zu dem zweiten Gerät übertragen werden soll, nicht durch die datenerzeugende Schaltung beschränkt.

Der Umschalter wird ausschließlich von der Kommunikationseinheit betätigt. Benötigt die Kommunikationseinheit Zugriff auf den Übergabespeicher, beispielsweise um auf Anforderung des zweiten Geräts von der datenerzeugenden Schaltung gesammelte medizinisch relevanten Daten zur Verfügung zu stellen, betätigt die Kommunikationseinheit den Umschalter und versetzt ihn dadurch aus seinem ersten Schaltzustand in den zweiten Schaltzustand. Nachdem die Kommunikationseinheit die benötigten Daten aus dem Übergabespeicher ausgelesen hat und deshalb vorläufig kein weiteren Zugriff auf den Übergabespeicher benötigt, betätigt die Kommunikationseinheit den Umschalter erneut und setzt ihn dadurch zurück in den ersten Schaltzustand, in dem nur die datenerzeugende Schaltung Zugriff auf den Speicher hat. Möglich ist es auch, den Übergabeschalter so auszuführen, dass er selbsttätig in den ersten Schaltzustand zurückfällt, wenn die Kommunikationseinheit für eine vorgegebene Anzahl von Taktzyklen nicht mehr auf den Übergabespeicher zugegriffen hat.

Da der Umschalter ohne Kommunikation mit der datenerzeugenden Schaltung zwischen dem ersten und dem zweiten Schaltzustand wechselt, können Schreib- und Lesevorgänge der datenerzeugenden Schaltung unterbrochen und somit gestört werden. Die von der datenerzeugenden Schaltung in den Übergabespeicher geschriebenen Daten können deshalb korrumpiert, unvollständig oder fehlerhaft sein. Dem damit verbundenen Problem kann beispielsweise dadurch begegnet werden, dass die datenerzeugende Schaltung einen Mikroprozessor enthält, der durch Umschaltvorgänge unterbrochene Schreib- bzw. Lesevorgänge wiederholt und über einen eigenen Speicher verfügt, in dem Daten vor Übertragung in den Übergabespeicher zwischengespeichert werden können. Bei einem unterbrochenen Schreibvorgang kann auf diese Weise der Speicher der datenerzeugenden Schaltung erneut ausgelesen und seinen Inhalt in den Übergabespeicher übertragen werden.

Eine weitere Möglichkeit, die sich insbesondere für Messgeräte eignet, die im Rahmen eines so genannten "continuous monitoring" in kurzen Zeitabständen von beispielsweise einer Minute Messdaten liefern, besteht darin, gescheiterte Schreibvorgänge zu ignorieren, da einzelne Messwerte oder sogar Messwertserien über mehrere Minuten, die durch unterbrochene Schreibvorgänge verloren gehen, bei einer späteren Auswertung interpoliert werden können.

Weitere Einzelheiten und Vorteile der Erfindung werden anhand von Ausführungsbeispielen unter Bezugnahme der beigefügten Zeichnungen erläutert. Die dabei beschriebenen Merkmale können einzeln und in Kombination zum Gegenstand von Ansprüchen gemacht werden. Gleiche und einander entsprechende Komponenten sind dabei mit übereinstimmenden Bezugszeichen gekennzeichnet. Es zeigen:
- Figur 1: eine schematische Darstellung eines Ausführungsbeispiels eines medizinischen Datenübertragungssystems beim Sammeln von medizinisch relevanten Daten;
- Figur 2: das in Figur 1 dargestellte Ausführungsbeispiel in einem zweiten Schaltzustand beim drahtlosen übertragen der medizinisch relevanten Daten;
- Figur 3: das dargestellte Ausführungsbeispiel beim drahtlosen Übertragen von Konfigurationsdaten;
- Figur 4: das dargestellte Ausführungsbeispiel in einem weiteren Schaltzustand nach der Übertragung von Konfigurationsdaten gemäß Figur 3;
- Figur 5: eine schematische Ausführung eines Ausführungsbeispiel der Datenbusarchitektur des dargestellten Datenübertragungssystems;
- Figur 6: ein weiteres Ausführungsbeispiel der Datenbusarchitektur des dargestellten Datenübertragungssystems; und
- Figur 7: ein weiteres Beispiel eines medizinischen Datenübertragungssystems.

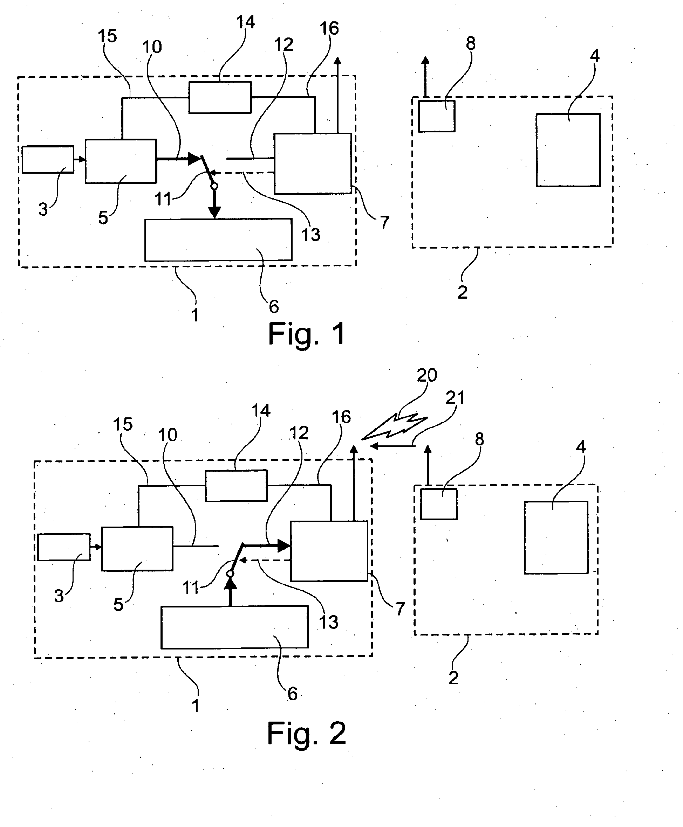

Das in Figur 1 dargestellte medizinische Übertragungssystem umfasst ein erstes Gerät 1, das bestimmungsgemäß von einem Patienten am Körper getragen wird und im Betrieb medizinisch relevante Daten erzeugt, die drahtlos zu einem zweiten Gerät 2 übertragen werden. Im dargestellten Ausführungsbeispiel handelt es sich bei dem ersten Gerät 1 um ein Messgerät mit einem implantierbaren Sensor 3 zur in-vivo Messung einer Analytkonzentration, beispielsweise der Glucosekonzentration, im Körper eines Patienten. Bei dem zweiten Gerät 2 handelt es sich um ein Anzeigegerät, das eine Ausgabeeinrichtung 4 zur Ausgabe von gemessenen Analytkonzentrationswerten, beispielsweise ein Display, aufweist.

Das erste Gerät 1 enthält eine Kommunikationseinheit 7, die einen Sender, einen Empfänger und einen Mikroprozessor zur Steuerung der Kommunikation mit dem zweiten Gerät 2 enthält. Ebenso enthält das zweite Gerät 2 eine Kommunikationseinheit 8, die einen Sender, einen Empfänger und einen Mikroprozessor zur Steuerung der Kommunikation mit dem ersten Gerät 1 enthält.

Die medizinisch relevanten Daten werden in dem ersten Gerät als Messdaten mittels einer Schaltung erzeugt, die neben dem Sensor 3 einen Mikroprozessor 5 enthält. Dieser Mikroprozessor 5 steuert die primäre Funktion der ersten Geräts 1, die bei dem dargestellten Beispiel das Erzeugen von Messdaten ist, aber beispielsweise auch in einer Behandlung des Patienten, beispielsweise durch Verabreichen eines Wirkstoffs, bestehen kann. Der Mikroprozessor 5 der datenerzeugenden Schaltung wird deshalb im Rahmen der Anmeldung auch als Systemprozessor bezeichnet. Die datenerzeugende Schaltung 3, 5 und die Kommunikationseinheit 7 sind jeweils über separate Versorgungsleitungen 15, 16 an eine Energiequelle 14, beispielsweise eine Batterie, angeschlossen. Diese Energiequelle 14 kann weder von der Daten erzeugenden Schaltung 3, 5 noch von der Kommunikationseinheit 7 abgeschaltet werden.

Bei dem dargestellten Ausführungsbeispiel werden analoge Sensorsignale, beispielsweise mit der gesuchten Messgröße korrelierende Stromstärken, mit einem Analogdigitalwandler des Mikroprozessors 5 digitalisiert und einer Vorauswertung unterzogen. Bei dieser Vorauswertung werden die digitalisierten Rohdaten des Sensors 3, die in ersten Zeitabständen von beispielsweise 1 Sekunde ermittelt werden, komprimiert und daraus Messdaten für zweite Zeitintervalle von beispielsweise 1 min erzeugt. Diese Datenkompression kann beispielsweise durch Mittelwertbildung erfolgen oder auch komplizierte Verfahren zur Datenaufbereitung oder Kompression nutzen, wie sie beispielsweise in der EP 1702559 A2 oder der DE 102004020160 A1 beschrieben sind.

Die von dem Mikroprozessor 5 erzeugten Messdaten werden in einem Übergabespeicher 6 abgelegt, an den der Mikroprozessor 5 über eine erste Datenleitung 10 angeschlossen ist. In dieser ersten Datenleitung 10 befindet sich ein Umschalter 11, der in Figur 1 in seinem ersten Schaltzustand dargestellt ist, in dem er die erste Datenleitung 10 schließt, so dass der datenerzeugende Mikroprozessor 5 Messdaten als medizinisch relevante Daten in den Übergabespeicher 6 schreiben kann.

Der Übergabespeicher 6 ist über eine zweite Datenleitung 12 an die Kommunikationseinheit 7 des ersten Geräts 2 angeschlossen. In dem in Figur 1 dargestellten ersten Schaltzustand des Umschalters 11 ist diese zweite Datenleitung 12 unterbrochen, so dass die Kommunikationseinheit 7 nicht auf den Übergabespeicher 6 zugreifen kann. Der Umschalter 11 wird bei dem dargestellten Ausführungsbeispiel ausschließlich von der Kommunikationseinheit 7, genauer gesagt dem in der Kommunikationseinheit 7 enthaltenen Mikroprozessor betätigt. Die Kommunikationseinheit 7 ist zu diesem Zweck über eine Steuerungsleitung 13 mit dem Umschalter verbunden. Bei Bedarf verschafft sich die Kommunikationseinheit 7 eine Zugriffsmöglichkeit auf den Übergabespeicher 6, indem sie diesen mittels eines Signals über die Steuerungsleitung 13 betätigt und aus dem in Figur 1 dargestellten ersten Schaltzustand in den in Figur 2 dargestellten zweiten Schaltzustand versetzt.

In dem zweiten Schaltzustand unterbricht der Umschalter 11 die erste Datenleitung 10 und schließt die zweite Datenleitung 12, so dass nun ausschließlich die Kommunikationseinheit 7 auf den Übergabespeicher 6 zugreifen und in ihm gespeicherte Messdaten auslesen kann. Diese Messdaten werden anschließend von der Kommunikationseinheit 7 drahtlos als Signalfolge 20 an das zweite Gerät 2 übertragen. Nach dem Auslesen des Übergabespeichers 6 betätigt die Kommunikationseinheit 7 den Umschalter 11 erneut, um ihn in seinen ersten Schaltzustand zurück zu versetzten, so dass der Übergabespeicher 11 wieder von der datenerzeugenden Schaltung, bei dem dargestellten Beispiel also dem an den Sensor 3 angeschlossenen Prozessor 5, mit Messdaten beschrieben werden kann. Ausgelesene Daten werden von der Kommunikationseinheit 7 in den Übergabespeicher 6 als gelesen markiert oder gelöscht, so dass die betreffenden Speicherplätze des Übergabespeichers 6 erneut beschrieben werden können.

Der Übergabespeicher 6 kann von der Daten erzeugenden Schaltung bzw. dem Systemprozessor 5 auch als Hauptspeicher genutzt werden. Möglich ist es aber auch, dass die Daten erzeugende Schaltung bzw. der Systemprozessor auf einen separaten Speicher zugreifen können und in dem Übergabespeicher ausschließlich zwischen der Daten erzeugenden Schaltung und der Kommunikationseinheit 7 auszutauschende Daten abgelegt werden.

Bei dem dargestellten Ausführungsbeispiel wird die Übertragung von Messdaten durch ein Anforderungssignal 21, das von der Kommunikationseinheit 8 des zweiten Geräts 2 gesendet wird, ausgelöst. Bei Empfang eines solchen Anforderungssignals 21 betätigt die Kommunikationseinheit 7 des ersten Geräts 1 den Umschalter 11, um den Übergabespeicher 11 auslesen zu können und sendet anschließend die gelesenen Messdaten. Von dem ersten Gerät 1 und von dem zweiten Gerät 2 gesendete Signalfolgen enthalten in der Regel stets eine charakteristische Kennung, die eine Beeinflussung durch systemfremde Geräte ausschließt. Für die Übertragung können beispielsweise paketorientierte Protokolle wie Bluetooth, Zigbee, HomeRF, Wibree, NFC, IEEE 802.11 oder andere Protokolle verwendet werden.

Da der Umschalter 11 ohne Kommunikation mit der datenerzeugenden Schaltung 3, 5 des ersten Geräts 1 zwischen seinen Schaltzuständen wechselt, können Schreibvorgänge der Schaltung bzw. des darin enthaltenen Mikroprozessors 5 unterbrochen und somit gestört werden. Als Folge eines unterbrochenen Schreibvorgangs können in dem Übergabespeicher 6 fehlerhafte Daten entstehen. Damit diese bei einer späteren Auswertung der Messdaten nicht zu einer Verfälschung der Ergebnisse führen, werden die Messdaten von dem Mikroprozessor 5 der Datenerzeugenden Schaltung in Datenblöcken zusammengefasst und zu den Datenblöcken jeweils eine Datensicherungssumme berechnet. Bei einem fehlerfreien, nicht unterbrochenen Schreibvorgang werden diese Datenblöcke zusammen mit ihrer jeweiligen Datensicherungssumme in dem Übergabespeicher 6 gespeichert. Durch Überprüfen dieser Datensicherungssumme kann später festgestellt werden, ob ein in dem Übergabespeicher 6 gespeicherter Datenblock fehlerhaft ist oder nicht. Die Datensicherungssumme kann von dem Mikroprozessor der Kommunikationseinheit 7 überprüft werden, so dass fehlerhafte Datenblöcke bereits frühzeitig erkannt und gar nicht erst zu dem zweiten Gerät 2 gesendet werden müssen. Möglich ist es aber auch, die Datensicherungssumme in einer Auswerteeinheit des zweiten Geräts 2 zu überprüfen und fehlerhafte Datensätze erst in dem zweiten Gerät 2 auszusortieren oder soweit möglich zu korrigieren. Bevorzugt wird die Datensicherungssumme sowohl von der Kommunikationseinheit 7 als auch von dem zweiten Gerät 2 überprüft. Beim Übertragungsvorgang werden für die entsprechenden Protokolle spezifische Sicherungssummen hinzugefügt.

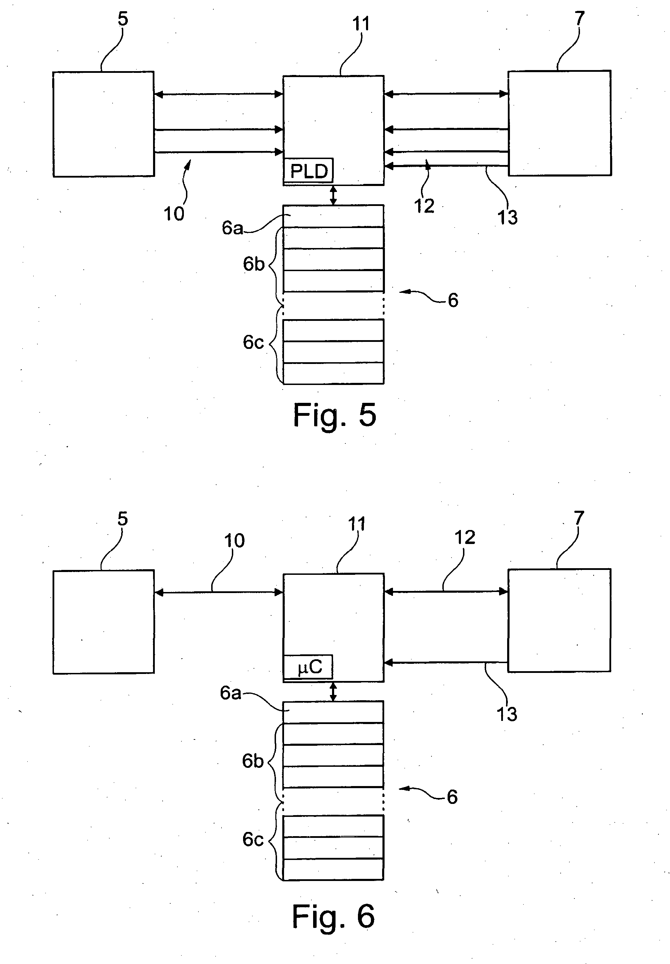

Bei dem beschriebenen Datenübertragungssystem können Daten auch von dem zweiten Gerät 2 zu dem ersten Gerät 1 übertragen werden, die dort von der datenerzeugenden Schaltung, insbesondere dem darin enthaltenen Mikroprozessor 5, benötigt werden. Bei derartigen Daten kann es sich beispielsweise um Konfigurationsdaten handeln, z. B. Werten der für den Sensor 3 optimalen Elektrodenspannung oder Steuerungsbefehlen, welche die Erzeugung medizinisch relevanten Daten betreffen. Die Übertragung derartiger Konfigurationsdaten von dem zweiten Gerät 2 zu dem ersten Gerät 1 wird als Download bezeichnet, um diesen Betriebsmodus von als Upload bezeichneten Übertragungen medizinisch relevanter Daten von dem ersten Gerät 1 zu dem zweiten Gerät 2 zu unterscheiden. Der in den Figuren 5 und 6 dargestellte Übergabespeicher 6 hat einen Header 6a, einen für Uploaddaten reservierten Speicherbereich 6b und ein für Downloaddaten reservierten Speicherbereich 6c.

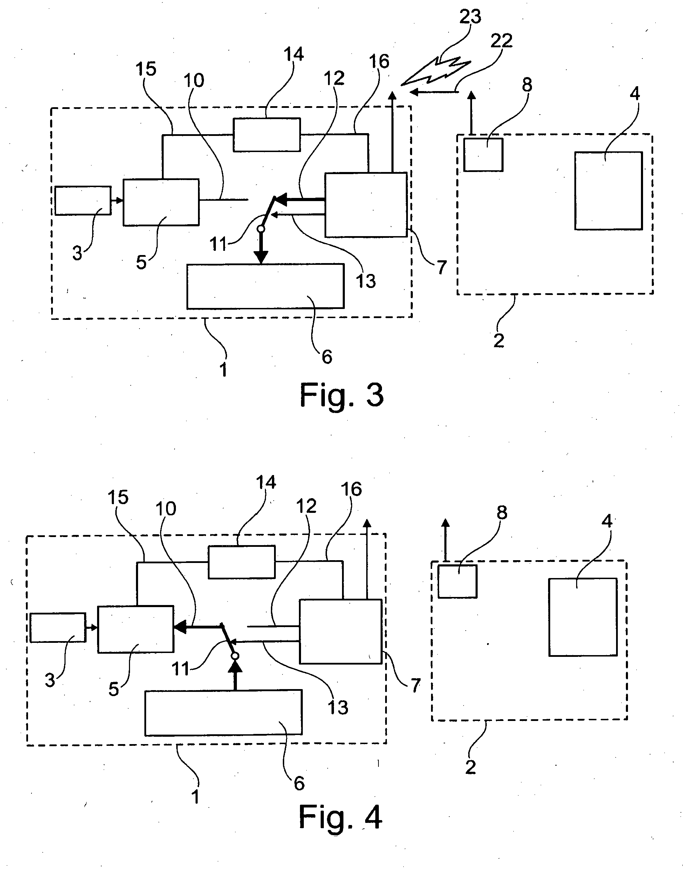

In dem Downloadmodus, der schematisch in Figur 3 dargestellt ist, sendet die Kommunikationseinheit 8 des zweiten Geräts 2 zunächst ein Anforderungssignal 22 und nachdem dessen Erhalt von der Kommunikationseinheit 7 des ersten Geräts betätigt wurde durch die Bezugszahl 23 dargestellte Daten, beispielsweise Konfigurationsdaten, die für die datenerzeugende Schaltung, bei dem dargestellten Beispiel also den an den Sensor 3 angeschlossenen Mikroprozessor 5 bestimmt sind. Die Kommunikationseinheit 7 versetzt daraufhin den Umschalter 11 in den zweiten Schaltzustand, so dass die Kommunikationseinheit 7 auf den Übergabespeicher 6 zugreifen und die empfangenen Daten dort speichern kann. Anschließend betätigt die Kommunikationseinheit den Umschalter 11 erneut, um ihn wieder in seinen ersten Schaltzustand zurück zu versetzten, in dem er an die datenerzeugende Schaltung, also an den darin enthaltenen Mikroprozessor 5, angeschlossen ist.

Da zwischen der datenerzeugenden Schaltung 3, 5 und der Kommunikationseinheit 7 nur über den Übergangsspeicher 6 Daten ausgetauscht werden, hat der Mikroprozessor 5 der datenerzeugenden Schaltung keine Kenntnis davon, ob für ihn neue Downloaddaten in dem Übergabespeicher 6 bereit gestellt wurden. Der Mikroprozessor 5 der datenerzeugenden Schaltung liest deshalb in festgelegten Zeitintervallen einen für Konfigurationsdaten und ähnliches reservierten Speicherbereich 6c des Übergabespeichers 6 aus. Um auszuschließen, dass Downloaddaten nur unvollständig von dem Systemprozessor 5 gelesen werden ist bei dem dargestellten Ausführungsbeispiel vorgesehen, dass die Kommunikationseinheit 7 nach einem abgeschlossenen Download den Umschalter 11 frühestens wieder in den zweiten Schaltzustand versetzt, nachdem eine Zeitspanne vergangen ist, die so groß ist, dass der Systemprozessor 5 zwischenzeitlich in jedem Fall den Downloadbereich des Übergabespeichers 6 ausgelesen hat, unabhängig davon, wann der Download erfolgt ist.

Beispielsweise kann die Wartezeit, in welcher der Mikroprozessor der Kommunikationseinheit 7 den Umschalter 11 nach einem abgeschlossenem Download nicht mehr betätigt, doppelt so groß wie der Zeitabstand zwischen den Versuchen des Systemprozessors 5 den Downloadbereich des Übergabespeichers 6 auszulesen, gewählt werden.

Die Datenleitungen 10, 12 zwischen dem Übergabespeicher 6 und der Kommunikationseinheit 7 bzw. zwischen dem Übergabespeicher 6 und dem Systemprozessor 5 können als Parallelbusse gemäß Figur 5 aufgebaut sein, also aus mehreren einzelnen Leitungen, beispielsweise etwa 30 Datenleitungen, bestehen. Bei dem dargestellten Ausführungsbeispiel sind 8 Bit Datenleitungen, 16 Bit Adressleitungen und 3 Bit Steuerleitungen vorgesehen. Bevorzugt schaltet der Umschalter 11 in einem solchen Fall alle einzelnen Leitungen einer solchen parallelen Datenleitung um. Möglich ist es aber auch, die Datenleitungen 10, 12 zwischen Übergabespeicher 6 und Kommunikationseinheit 7 bzw. Übergabespeicher 6 und Systemprozessor 5 seriell auszubilden. Geeignete Seriell-Bussysteme sind beispielsweise SPI oder ICC. Bei einer derartigen seriellen Ausbildung der Datenleitungen 10, 12 kann der Übergabespeicher 6 beispielsweise als EEPROM ausgebildet werden, was bei einer eventuellen Unterbrechung der Vorsorgungsspannung den Vorteil einer dauerhaften Zwischenspeicherung hat. Insbesondere bei einer parallelen Ausgestaltung der Datenleitungen 10, 12 kann Übergabespeicher aber beispielsweise auch als RAM-Speicher ausgebildet werden, die für Systeme mit schnellem Zugriff besonders vorteilhaft sind.

Der Umschalter 11 kann prinzipiell als ein mechanischer Schalter oder ein elektronischer Schalter, beispielsweise ein Transistorschalter realisiert werden. Insbesondere bei Ausgestaltung der Datenleitungen 10, 12 als Parallelbusse kann der Umschalter beispielsweise als ASIC oder sogar als Mikroprozessor ausgebildet werden, bzw. einen solchen enthalten. Daneben besteht auch die Möglichkeit, den Umschalter mit Logik- Bausteinen (AND, NAND, OR, NOR, EXOR, latches etc.) oder auch mittels einer freiprogrammierbaren Logik (PLD) zu realisieren.

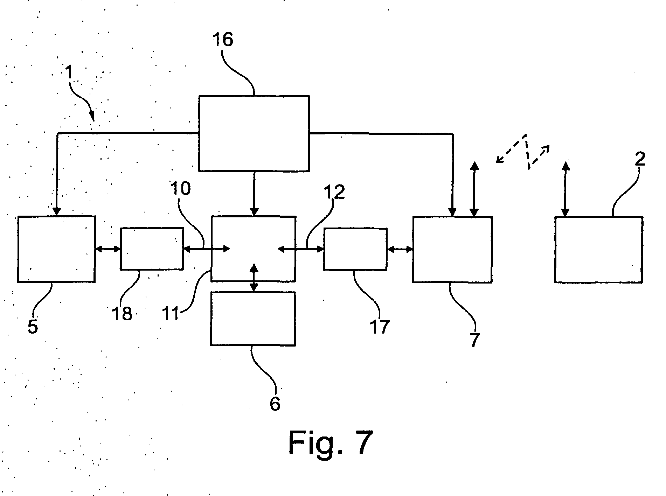

Figur 7 zeigt ein nicht der Erfindung entsprechendes Beispiel eines medizinischen Datenübertragungssystems, das sich von dem der Erfindung entsprechenden Datenübertragungssystem im wesentlichen dadurch unterscheidet, dass der Umschalter 11 von einem Random Access Prozessor 17 betätigt wird, so dass der Systemprozessor 5 der Daten erzeugenden Schaltung und die Kommunikationseinheit 7 jeweils abwechselnd auf den Übergabespeicher 11 zugreifen können. Die Umschaltzeitpunkte können dabei von dem Random Access Prozessor 17 beliebig gewählt werden, beispielsweise durch einen internen Zufallsgenerator vorgegeben werden.

Der Systemprozessor 5 und die Kommunikationseinheit 11 haben jeweils Zugriff auf einen Zwischenspeicher 17, 18, in dem die für den Übergabespeicher 6 bestimmten Daten zwischengespeichert werden können.

Das beschriebene Verfahren zur Datenübertragung kann für drahtgebundene und drahtlose, beispielsweise auch optische, Datenübertragung verwendet werden. Der Umschalter 11 kann in entsprechender Weise auch in dem Gerät 2 zwischen der Kommunikationseinheit 8 und der Ausgabeeinheit 4 eingesetzt werden. Es kann in dem ersten und in dem zweiten Gerät jeweils ein Umschalter 11 eingebaut sein.

### Bezugszahlenliste

- 1: erstes Gerät
- 2: zweites Gerät
- 3: Sensor
- 4: Ausgabeeinrichtung
- 5: Mikroprozessor
- 6: Übergabespeicher
- 6a: Header
- 6b: Speicherbereich
- 6c: Speicherbereich
- 7: Kommunikationseinheit
- 8: Kommunikationseinheit
- 10: erste Datenleitung
- 11: Umschalter
- 12: zweite Datenleitung
- 13: Steuerungsleitung
- 14: Energiequelle
- 15: Versorgungsleitung
- 16: Versorgungsleitung
- 20: Signalfolge
- 21: Anforderungssignal
- 22: Anforderungssignal
- 30: erstes Gerät
- 31: zweites Gerät
- 32: Mikroprozessor
- 33: Schalter
- 34: Frequenzgenerator
- 35: Antenne
- 36: Prozessor
- 37: Empfänger

## Patentansprüche

1. Medizinisches Datenübertragungssystem mit einem ersten Gerät (1), das bestimmungsgemäß von einem Patienten am Körper getragen wird und im Betrieb medizinisch relevante Daten erzeugt, die drahtlos zu einem zweiten Gerät (2) übertragen werden, wobei
das erste Gerät (1) eine Kommunikationseinheit (7) zur drahtlosen Kommunikation mit dem zweiten Gerät (2), eine Schaltung (3, 5), die im Betrieb die medizinisch relevante Daten erzeugt, und einen Übergabespeicher (6) zur Zwischenspeicherung von Daten, die von der Schaltung (3, 5) zu der Kommunikationseinheit (7) oder von der Kommunikationseinheit (7) zu der Schaltung (3, 5) transferiert werden sollen, enthält, und wobei die datenerzeugende Schaltung (3, 5) über eine erste Datenleitung (10) an den Übergabespeicher (6) angeschlossen ist und die Kommunikationseinheit (7) über eine zweite Datenleitung (12) an den Übergabespeicher (6) angeschlossen ist,
wobei
das erste Gerät (1) einen Umschalter (11) enthält, der dazu eingerichtet ist in einem ersten Schaltzustand die erste Datenleitung (10) zu schließen und die zweite Datenleitung (12) zu unterbrechen und in einem zweiten Schaltzustand die erste Datenleitung (10) zu unterbrechen und die zweite Datenleitung (12) zu schließen, und
der Umschalter (11) ein mechanischer oder elektronischer Schalter ist,
**dadurch gekennzeichnet, dass**
der Umschalter (11) sich in der ersten Datenleitung (10) befindet,
zwischen der datenerzeugenden Schaltung (3, 5) und der Kommunikationseinheit (7) nur über den Übergabespeicher (6) Daten ausgetauscht werden,
die Kommunikationseinheit (7) über eine Steuerungsleitung (13) mit dem Umschalter (11) verbunden ist, und das Datenübertragungssystem eingerichtet ist, mittels dieser Steuerungsleitung (13) den Umschalter (11) ausschließlich von einem in der Kommunikationseinheit (7) enthaltenen Mikroprozessor zu betätigen, um so zwischen dem ersten und dem zweiten Schaltzustand zu wechseln.

2. Datenübertragungssystem nach einem der vorhergehenden Ansprüche, **dadurch gekennzeichnet, dass** die datenerzeugende Schaltung (3, 5) einen Mikroprozessor (5) zur Steuerung der Datenerzeugung enthält.

3. Datenübertragungssystem nach einem der vorhergehenden Ansprüche, **dadurch gekennzeichnet, dass** die datenerzeugende Schaltung (3, 5) einen Sensor (3) enthält, der im Betrieb am oder im Körper eines Patienten einen medizinisch relevanter Parameter, insbesondere eine Analytkonzentration, misst und in dem Übergabespeicher (6) Messdaten des Sensors (3) speichert.

4. Datenübertragungssystem nach Anspruch 3, **dadurch gekennzeichnet, dass** das zweite Gerät (2) eine Anzeigeeinrichtung zum Anzeigen von Messergebnissen des Sensors (3) aufweist.

5. Datenübertragungssystem nach einem der vorhergehenden Ansprüche, **dadurch gekennzeichnet, dass** die Schaltung (3, 5) die von ihr erzeugten Daten in Datenblöcken zusammenfasst, zu den Datenblöcken jeweils eine Datensicherungssumme berechnet, um die Datenblöcke zusammen mit der dazugehörenden Datensicherungssumme in dem Übergabespeicher (6) zu speichern.

6. Datenübertragungssystem nach Anspruch 5, **dadurch gekennzeichnet, dass** die Kommunikationseinheit (7) die Datensicherungssumme von aus dem Übergabespeicher (6) gelesenen Datenblöcken überprüft und nur Datenblöcke mit einer korrekten Datensicherungssumme aussendet.

## Claims

1. Medical data transmission system comprising a first device (1) which is designed to be worn on the body by a patient and which generates medically relevant data when in operation, said data being transmitted to a second device (2) in a wireless manner, wherein
the first device (1) comprises a communication unit (7) for wireless communication with the second device (2), a circuit (3, 5) which, in operation, generates medically relevant data, and a handover memory (6) for intermediate storage of data that are to be transmitted from the circuit (3, 5) to the communication unit (7) or from the communication unit (7) to the circuit (3, 5), and wherein the data-generating circuit (3, 5) is connected to the handover memory (6) via a first data line (10) and the communication unit (7) is connected to the handover memory (6) via a second data line (12),
wherein
the first device (1) contains a changeover switch (11) configured to close the first data line (10) and interrupt the second data line (12) in a first switching state, and to interrupt the first data line (10) and to close the second data line (12) in a second switching state, and
the changeover switch (11) is a mechanical or an electronic switch,
**characterized in that**
the changeover switch (11) is arranged in the first data line (10),
data are exchanged between the data-generating circuit (3, 5) and the communication unit (7) only via the handover memory (6),
the communication unit (7) is connected to the changeover switch (11) via a control line (13), and the data transmission system being configured to actuate the changeover switch (11) by means of this control line (13) exclusively from a microprocessor contained in the communication unit (7) in order to thereby switch between the first and the second switching state.

2. Data transmission system according to any one of the preceding claims, **characterized in that** the data-generating circuit (3, 5) contains a microprocessor (5) for controlling the generation of data.

3. Data transmission system according to any one of the preceding claims, **characterized in that** the data-generating circuit (3, 5) contains a sensor (3) which, in operation on or in the body of a patient, measures a medically relevant parameter, in particular an analyte concentration, and stores measuring data of the sensor (3) in the handover memory (6).

4. Data transmission system according to claim 3, **characterized in that** the second device (2) has a display facility for displaying measuring results of the sensor (3).

5. Data transmission system according to any one of the preceding claims, **characterized in that** the circuit (3, 5) combines the data it generates into data blocks, calculates a data protection sum each for the data blocks in order to store the data blocks together with the corresponding data protection sum in the handover memory (6).

6. Data transmission system according to claim 5, **characterized in that** the communication unit (7, 8) checks the data protection sum of data blocks that are read-out from the handover memory (6) and sends only data blocks that have a correct data sum.

## Revendications

1. Système médical de transmission de données avec un premier appareil (1), qui est porté au niveau du corps par un patient conformément aux instructions et génère en service des données pertinentes sur le plan médical, lesquelles sont transmises sans fil à un deuxième appareil (2), dans lequel
le premier appareil (1) contient une unité de communication (7) pour une communication sans fil avec le deuxième appareil (2), un circuit (3, 5) qui génère les données pertinentes sur le plan médical, et une mémoire de transfert (6) pour la mise en mémoire intermédiaire de données qui doivent être transférées du circuit (3, 5) à l'unité de communication (7) ou de l'unité de communication (7) au circuit (3, 5), et dans lequel le circuit générateur de données (3, 5) est raccordé par l'intermédiaire d'une première ligne de transmission de données (10) à la mémoire de transfert (6) et l'unité de communication (7) est raccordée à la mémoire de transfert (6) par l'intermédiaire d'une deuxième ligne de transmission de données (12),
dans lequel
le premier appareil (1) contient un commutateur (11), qui est conçu pour fermer la première ligne de transmission de données (10) dans un premier état de commutation et pour interrompre la deuxième ligne de transmission de données (12) et pour interrompre la première ligne de transmission de données (10) dans un deuxième état de commutation et pour fermer la deuxième ligne de transmission de données, et
le commutateur (11) est un interrupteur mécanique ou électronique, **caractérisé en ce que**
le commutateur (11) se trouve dans la première ligne de transmission de données (10),
entre le circuit générateur de données (3, 5) et l'unité de communication (7) des données ne sont échangées que par l'intermédiaire de la mémoire de transfert (6),
l'unité de communication (7) est reliée au commutateur (11) par l'intermédiaire d'une ligne de commande (13), et le système de transmission de données est conçu pour actionner le commutateur (11) au moyen de cette ligne de commande (13) exclusivement à partir d'un microprocesseur contenu dans l'unité de commande (7), afin de passer du premier au deuxième état de commutation.

2. Système de transmission de données selon l'une des revendications précédentes, **caractérisé en ce que** le circuit générateur de données (3, 5) contient un microprocesseur (5) pour la commande de la génération de données.

3. Système de transmission de données selon l'une des revendications précédentes, **caractérisé en ce que** le circuit générateur de données (3, 5) contient un capteur (3) qui, en service, mesure sur ou dans le corps d'un patient un paramètre pertinent sur le plan médical, en particulier la concentration en analytes, et met en mémoire les données de mesure du capteur (3) dans la mémoire de transfert (6).

4. Système de transmission de données selon la revendication 3, **caractérisé en ce que** le deuxième appareil (2) présente un dispositif d'affichage pour l'affichage des résultats de mesure du capteur (3).

5. Système de transmission de données selon l'une des revendications précédentes, **caractérisé en ce que** le circuit (3, 5) rassemble les données qu'il a générées dans des blocs de données, calcule pour les blocs de données respectivement une somme de sauvegarde de données afin de mettre en mémoire les blocs de données conjointement avec la somme de sauvegarde de données correspondante dans la mémoire de transfert (6).

6. Système de transmission de données selon la revendication 5, **caractérisé en ce que** l'unité de communication (7) vérifie la somme de sauvegarde de données à partir des blocs de données lus dans la mémoire de transfert (6) et envoie uniquement les blocs de données ayant une somme de sauvegarde de données correcte.
